# EUROPEAN PATENT APPLICATION

(11) **EP 2 774 577 A2**
(43) Date of publication of application: **10.09.2014**
(21) Application number: 14158364.1
(22) Date of filing: 07.03.2014
(51) Int. Cl.: A61C 13/00, A61B 19/00

(54) **Method and system for dental implant path planning**

(30) Priority: 07.03.2013 TW 102108117
(71) Applicant: National Cheng Kung University, Tainan City 701 (TW); CHOICE Biotech Inc., Tainan City 744 (TW)
(72) Inventor: Fang, Jing-Jing, 701 Tainan City (TW); Leong, Iat-Fai, 701 Tainan City (TW)
(74) Representative: Von Kreisler Selting Werner - Partnerschaft von Patentanwälten und Rechtsanwälten mbB

(57) **Abstract**

A method for dental implant path planning is applied to a computer displaying a planning interface having a jaw image zone, a cross-section image zone and a reference zone, and comprises steps of: displaying a jaw image in the jaw image zone; generating a dental arch according to the jaw image; labeling an implant position in the jaw image; lapping an implant image over the jaw image according to the implant position; displaying at least two cross-section images in the cross-section image zone according to the implant image and the jaw image; displaying a plurality of characteristic values in the reference zone according to a plurality of characteristic distances in the cross-section images; and adjusting the position of the implant image relative to the jaw image by referring to the cross-section images or the characteristic values.

## Description

### BACKGROUND OF THE INVENTION

### Field of Invention

The invention relates to a method and system for dental implant path planning.

### Related Art

The dental implantation is implanting a dental implant (such as a titanic artificial tooth root) onto the alveolar bone of the mouth for replacing the original tooth root, and the implant and bone will be combined together after 3-6 months. Then, an artificial tooth can be disposed onto the implant, and thereby the user can get a beautiful oral appearance and mastication function back.

Clinically, there are many things to be done before the dental implantation surgery, such as analyzing the user's conditions for the dental implant, determining if the bone addition is required or not in consideration of the bone thickness, determining the implanted position of the dental implant, and knowing the geometric relationship between the implant and the related tissues. The above things are also related to the recovery of mastication function and a beautiful oral look. More importantly, they are also related to the safety of the dental implantation surgery. Accordingly, in order to reduce the surgery risk and increase the success rate of the surgery, good auxiliary instruments and surgery planning are required.

In general, a boring guiding plate is made to assist the surgeon to accurately implant the dental implant on the jawbone. A reliable boring guiding plate is the key to the success of the implantation. For the conventional dental implantation, the x-rays of full mouth are obtained by the full-mouth x-ray photography. By referring to the x-rays, the surgeon can roughly plan the location of the implant, determine the implanted position on a plaster dental cast and make the boring guiding plate. Apparently, this kind of surgery planning by the x-ray photography has a serious problem of lacking accuracy because the implantation model is constructed from two-dimensional images, considerably relying on the professional determination and experiences of the surgeon.

With the progress of science and the technology of computer hardware, the computed tomography (CT) images and simulation software can provide the surgeon a three-dimensional model of the patient's jaw and mouth. By such advantage, implementing the surgery planning by computer software and thereby making the boring guiding plate becomes a popular field of the dental market. However, although the observation by three-dimensional images can assist the surgery planning, some data, such as characteristic lengths or angles, still need to be determined and inputted to the software manually. In other words, the professional knowledge has not been computerized and introduced to the auxiliary assistance.

Therefore, it is an important subject to make the surgeon easily and accurately plan the required dental implant path for the individual by effectively using the computer simulation technology with the help of the introduction of the professional knowledge so that the boring guiding plate can be made with customization.

### SUMMARY OF THE INVENTION

In view of the foregoing subject, an objective is to provide a system and method for dental implant path planning, and by operating the system or implementing the method on computer, the surgeon can safely and effectively use the computer simulation technology with the help of the professional knowledge to plan the customized implant path and make the customized boring guiding plate.

Besides, a planning interface for dental implant path planning is also disclosed in this invention, which can display visualized and quantified data so that the surgeon can easily refer to the professional knowledge on the surgery planning.

To achieve the above objective, a method for dental implant path planning according to the invention is applied to a computer displaying a planning interface having a jaw image zone, a cross-section image zone and a reference zone, and comprises steps of: displaying a jaw image in the jaw image zone; generating a dental arch according to the jaw image; labeling an implant position in the jaw image; lapping an implant image over the jaw image according to the implant position; displaying at least two cross-section images in the cross-section image zone according to the implant image and the jaw image; displaying a plurality of characteristic values in the reference zone according to a plurality of characteristic distances in the cross-section images; and adjusting the position of the implant image relative to the jaw image by referring to the cross-section images or the characteristic values.

To achieve the above objective, a system for dental implant path planning according to the invention comprises a display module, a processing module, a memory module, and at least a program stored in the memory module and executed by the processing module. The program comprises: a command for displaying a jaw image in a jaw image zone; a command for generating a dental arch according to the jaw image; a command for labeling an implant position in the jaw image; a command for lapping an implant image over the jaw image according to the implant position; a command for displaying at least two cross-section images in a cross-section image zone according to the implant image and the jaw image; a command for displaying a plurality of characteristic values in a reference zone according to a plurality of characteristic distances in the cross-section images; and a command for adjusting the position of the implant image relative to the jaw image by referring to the cross-section images or the characteristic values.

### BRIEF DESCRIPTION OF THE DRAWINGS

The invention will become more fully understood from the detailed description and accompanying drawings, which are given for illustration only, and thus are not limitative of the present invention, and wherein:
FIG. 1 is a block diagram of a system for dental implant path planning according to a preferred embodiment of the invention;
FIG. 2 is a schematic diagram of a planning interface of the system for dental implant path planning according to a preferred embodiment of the invention;
FIGS. 3A-3F are schematic diagrams showing the execution of the all commands captured from the planning interface of this invention;
FIG. 3G is a schematic diagram of characteristic distances in the buccal-lingual (BL) cross-section image in this invention;
FIG. 3H is a schematic diagram of the characteristic distances in the mesial-distal (MD) cross-section image in this invention; and
FIG. 4 is a flow chart of a method for dental implant path planning according to a preferred embodiment of the invention.

### DETAILED DESCRIPTION OF THE INVENTION

The present invention will be apparent from the following detailed description, which proceeds with reference to the accompanying drawings, wherein the same references relate to the same elements.

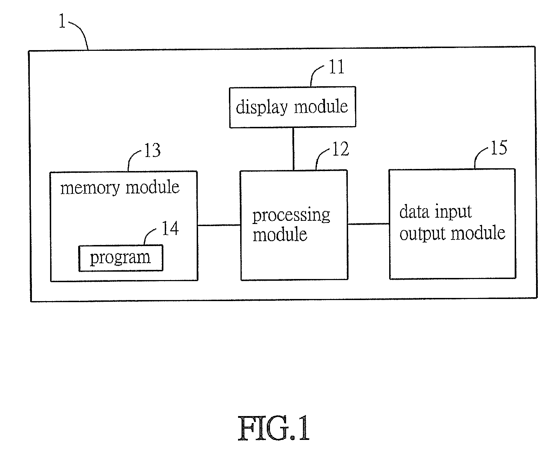

FIG. 1 is a block diagram of a system for dental implant path planning according to a preferred embodiment of the invention. As shown in FIG. 1, the system 1 for dental implant path planning includes a display module 11, a processing module 12, a memory module 13 and one or more programs 14.

The processing module 12 is coupled to the display module 11 and the memory module 13. The processing unit 12 is a central processing unit (CPU) for example, mainly managing calculation and processing. The display module 11 can include a displayer and a graphic processing unit. The displayer is a liquid crystal display (LCD) panel or an organic light emitting diode (OLED) display panel for example, and can process signals to display images for the user to view. The memory module 13 can include volatile memory or non-volatile memory. For example, the volatile memory is random access memory (RAM), and the non-volatile memory is flash memory. The memory module 13 can store an operating system, programs 14 and other application programs.

Besides, the system 1 also includes a data input output module 15, which can connect to periphery appliances for the data input and output. Specifically, the data input output module 15 is an optical disk driver or a USB connector for example. In other embodiments, the data input and output also can be implemented by a communication module (pertaining to wired or wireless network).

To be noted, the operator of the system 1 for dental implant path planning can be a surgeon, a technical person in a medical team, or a member of a company providing the service of dental implantation.

In this embodiment, the system 1 just executes a program 14 for the surgery planning for example, but the invention is not limited thereto however. In other embodiments, a plurality of commands can be respectively loaded to the corresponding programs.

The program 14 is stored in the memory module 13 and can be executed by the processing module 12. The program 14 includes many commands as follows:
1. a display command for displaying a jaw image in a jaw image zone;
2. an arch generation command for generating a dental arch according to the jaw image;
3. a label command for labeling an implant position in the jaw image;
4. an image overlap command for lapping an implant image over the jaw image according to the implant position;
5. a cross-section generation command for displaying at least two cross-section images in a cross-section image zone according to the implant image and the jaw image;
6. a value display command for displaying a plurality of characteristic values in a reference zone according to a plurality of characteristic distances in the cross-section images; and
7. an adjustment command for adjusting the position of the implant image relative to the jaw image by referring to the cross-section images or the characteristic values.

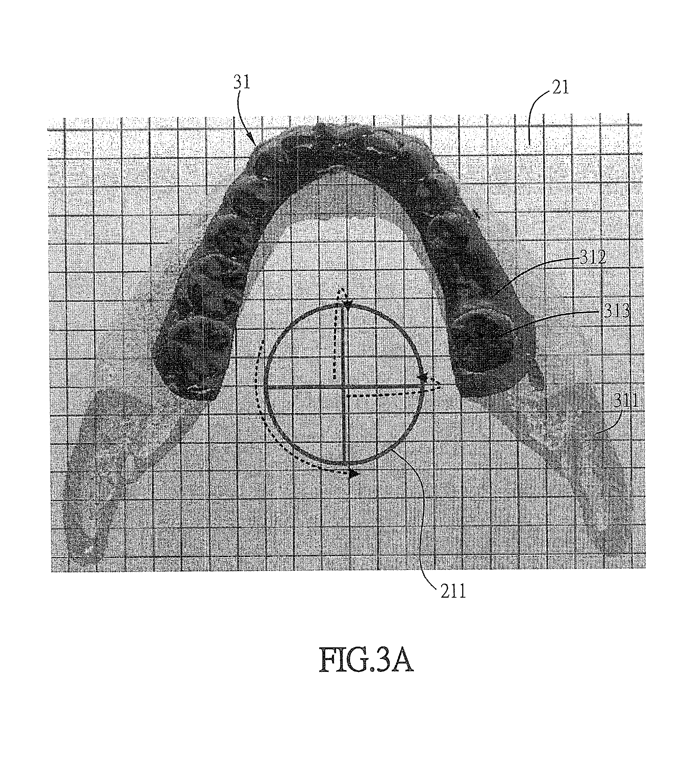

FGI. 2 is a schematic diagram of a planning interface of the system for dental implant path planning, and FIGS. 3A-3E are schematic diagrams showing the execution of the all commands captured from the planning interface. The following is the further illustration of the all commands by referring to FIG.S. 2 and 3A-3H.

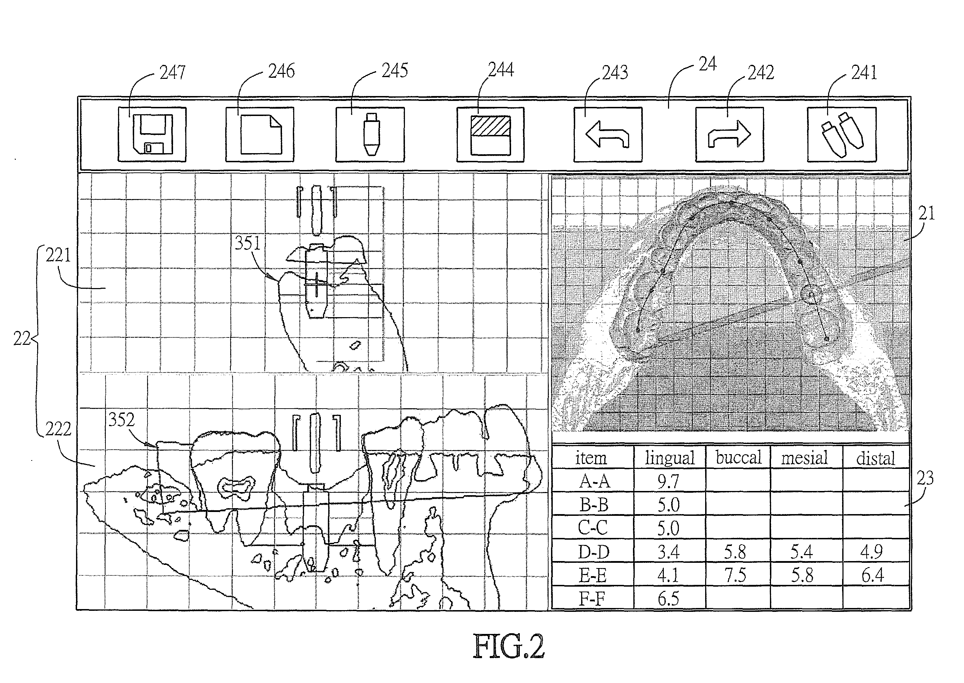

As shown in FIG. 2, the planning interface 2 of the system 1 is displayed by the display module 11, and includes a jaw image zone 21, a cross-section image zone 22, a reference zone 23 and a function item zone 24. The function item zone 24 displays a plurality of operating units 241-247, and when an operator selects one of them, the system 1 can execute the corresponding function. For example, selecting or clicking the operating unit 247 can store data, selecting or clicking the operating unit 246 can load data, and selecting or clicking the operating unit 243 can go back to the previous step. The cross-section image zone 22 can be divided into a buccal-lingual (BL) cross-section image unit 221 and a mesial-distal (MD) cross-section image unit 222.

As shown in FIG. 3A, when the command of displaying the jaw image in the jaw image zone is executed, the jaw image 31 will be displayed in the jaw image zone 21 (FIG. 3A is slightly different from FIG. 2 because on different command stages). The jaw image 31 can be stored in the memory module 13 or inputted through the data input output module 15. In general, the jaw image 31 is a three-dimensional image, and can be obtained by photographing the patient by using noninvasive medical photography. The jaw image 31 includes a computed tomography (CT) image of upper jaw or lower jaw for example. The jaw image 31 can show, for example, an upper jaw, a lower jaw, teeth, artificial labels, blood vessels, nerves, a tooth root, a titanic ring on the occlusal splint, or implant. The jaw image 31 can be a three-dimensional image of upper or lower jaw, and here FIG. 3A shows a lower jaw for example, and the jaw image 31 apparently shows a jawbone portion 311, a gums portion 312 and a tooth portion 313.

However, the jaw image 31 is obtained preferably by the following method.

A plaster dental cast of a patient is made and then scanned to obtain the image of upper and lower jaws. Besides, the patient wearing the occlusal splint is made undergo a CT scan to obtain the CT image. Then, the image model of the jaw bone, teeth, gums, and labels on the occlusal splint can be reconstructed by using the image of upper and lower jaws and the CT image, wherein the two kinds of images can be matched together by the labels. Finally, the jaw image 31 can be obtained after the image of upper and lower jaws replaces the teeth portion of the CT image.

The jaw image zone 21 also can show a control label 211, and the user can operate an external input appliance to drag or click the label 211 for changing the visual angle of the jaw image 31. For example, when clicking the left mouse button and dragging the label 211, the user can view the rotation of the jaw image 31. When clicking the right mouse button and dragging the label 211, the user can move the jaw image 31. When clicking the label 211 and scrolling the mouse wheel, the user can zoom in or zoom out the jaw image 31.

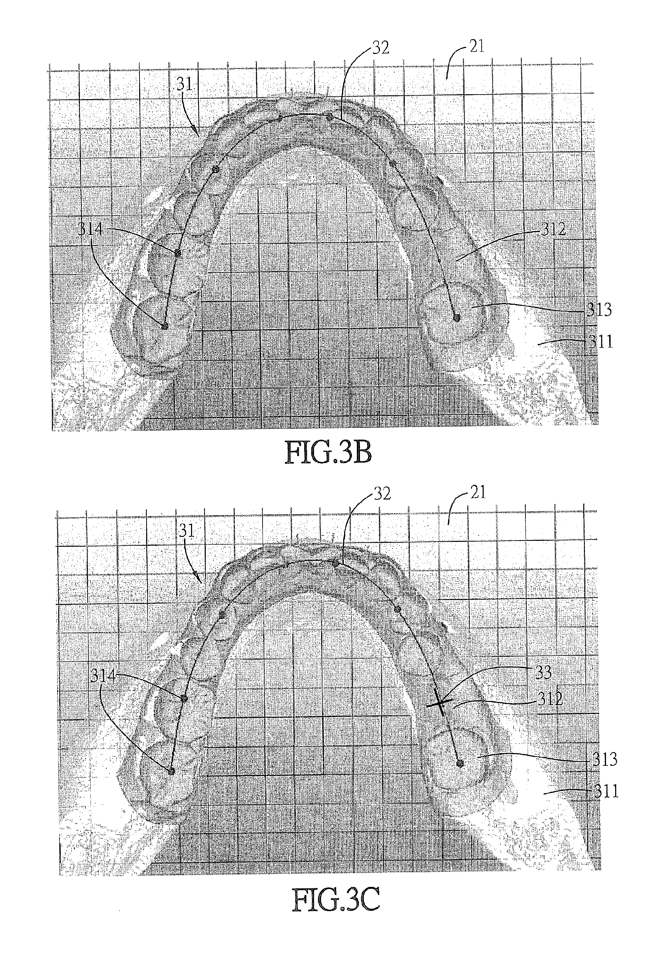

As shown in FIG. 3B, when the command of generating the dental arch according to the jaw image is executed, the program 14 will automatically detect the buccal-lingual (BL) tooth tips and trenches of a plurality of tooth portions 313 in the jaw image 31 and generate a plurality of crown labels 314 thereon. Subsequently, the program 14 can allow the user to manually connect the crown labels 1314 with a line, or preferably, the program 14 automatically connects the crown labels 314, for generating the dental arch 32. Not only the program 14 automatically generates the crown labels 314, the user also can manually make the crown labels 314 for generating the dental arch 32. Besides, for more flexibility, the user can add or withdraw the crown labels 314. Moreover, after generating the dental arch 32, the user can execute the command of adjusting the dental arch 32. For example, the user can adjust the positions of the crown labels 314 or the curvature of the dental arch 32 in order to adjust the dental arch 32.

As shown in FIG. 3C, when the command of labeling the implant position in the jaw image is executed, the user can operate an external input appliance and click at a proper position to set a position label 33 in the jaw image 31 to label the implant position for determining the location of the implant of the surgery planning, i.e. the main location of the dental implant path. In other embodiments, if there are many implants to be implanted in the surgery planning, a plurality of implant positions can be set. In this embodiment, the position label 33 is a cross sign for example, but however this invention is not limited thereto.

In addition to the manual setting, the implant position also can be determined according to the limit position of the cross-section of the jaw bone on the patient's dynamic occlusal surface. If the dynamic occlusal surface can not be generated, the implant position also can be determined through the inertia axis of the cross-section of the jaw bone. However, the invention is not limited thereto.

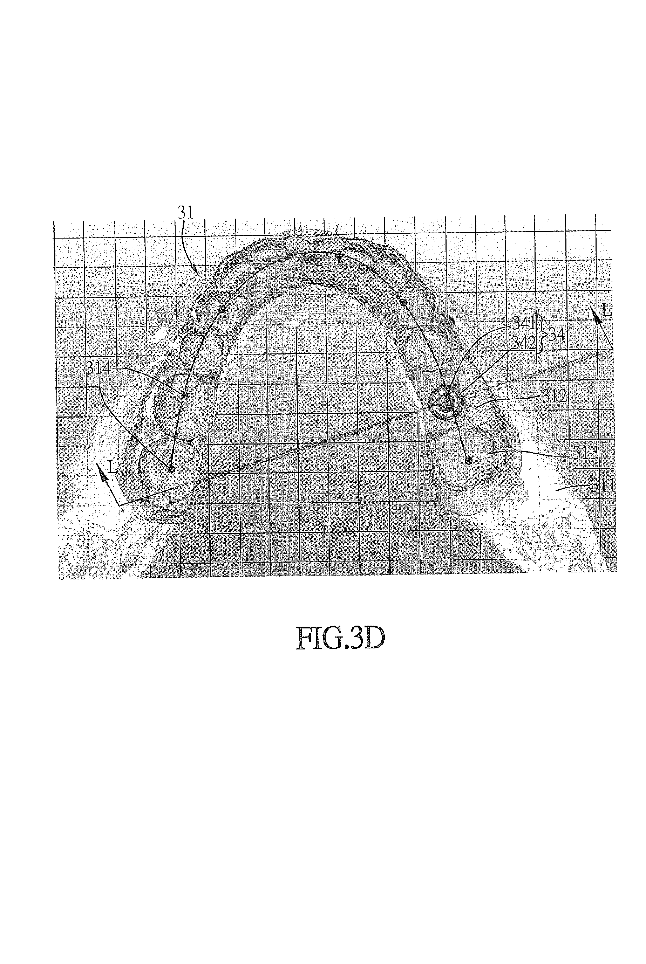

As shown in FIG. 3D, when the command of lapping the implant image over the jaw image according to the implant position is executed, the program 14 will load and generate the selected implant image 34 according to the setting, and obtain the coordinates of the position label 33 in the jaw image zone 21 for lapping the implant image 34 over the position label 33 of the jaw image 31.

When the implant image 34 is disposed, not only the entering point of the implant image 34 relative to the jaw image 31 is determined, but also the terminal point of the implant image 34 relative to the jaw image 31 needs to be determined. For determining the terminal point, the nerves and blood vessels of the upper and lower jaws need to be avoided, and the safety distance from the bone wall or adjacent tooth root also needs to be kept. In detail, the image recognition technology can be used to determine the positions of the jaw bone, nerves and teeth in the jaw image 31 and to set the safety distance of the nerve area. For the lower jaw as an example, a safety height of the terminal point is 2mm above the nerve's edge, and a safety implant orientation is defined as an interval between the implant and the bone wall.

To be noted, the implant image 34 can be generated by selecting from a plurality of default implant modules or by setting a plurality of implant parameters such as including the length or outer diameter of the implant. Besides, the implant image 34 can be divided into an implant body portion 341 and a metal ring portion 342 (such as a titanic ring of the occlusal splint required for the surgery), and the central axes of the portions 341 and 342 both lie in the direction of the central axis of the implant image 34, so that when one of them is adjusted in orientation, the other can change accordingly. Thereby, the implant, a rigid short cylinder, can be harmoniously related to the titanic ring in orientation because limited by the titanic ring, when the implantation is performed by the titanic ring passing through the occlusal splint.

When the command of displaying at least two cross-section images in the cross-section image zone according to the implant image and the jaw image is executed, the program 14 will cause the cross-section images of the implant position 32 where the implant image 34 is lapped over the jaw image to be displayed in the cross-section image zone 22. In FIG. 2, a buccal-lingual (BL) cross-section image 351 and a mesial-distal (MD) cross-section image 352 are shown, and preferably they are perpendicular to each other in visual angle for highlighting the characteristics passing through the jaw image 31. In detail, the BL cross-section image 351 and MD cross-section image 352 generated by the program 14 are taken along the section L-L (as shown in FIG. 3D) that passes through the central axis of the implant image 34 and is perpendicular to the tangent line of the dental arch 32 at the position label 33. In other embodiments, the program 14 can allow the user to adjust the angle of the section that may be not perpendicular to the dental arch 32. Otherwise, other different cross-section images can be generated.

As an example, the tissues or artificial objects can be seen in the BL cross-section image 351, including the bone wall, soft tissues, metal ring, implant body, etc. More importantly, the user or surgeon can directly observe the relative position and distance among the selected implant body portion 341, tissues and metal ring portion 342 to simulate the condition of the surgery.

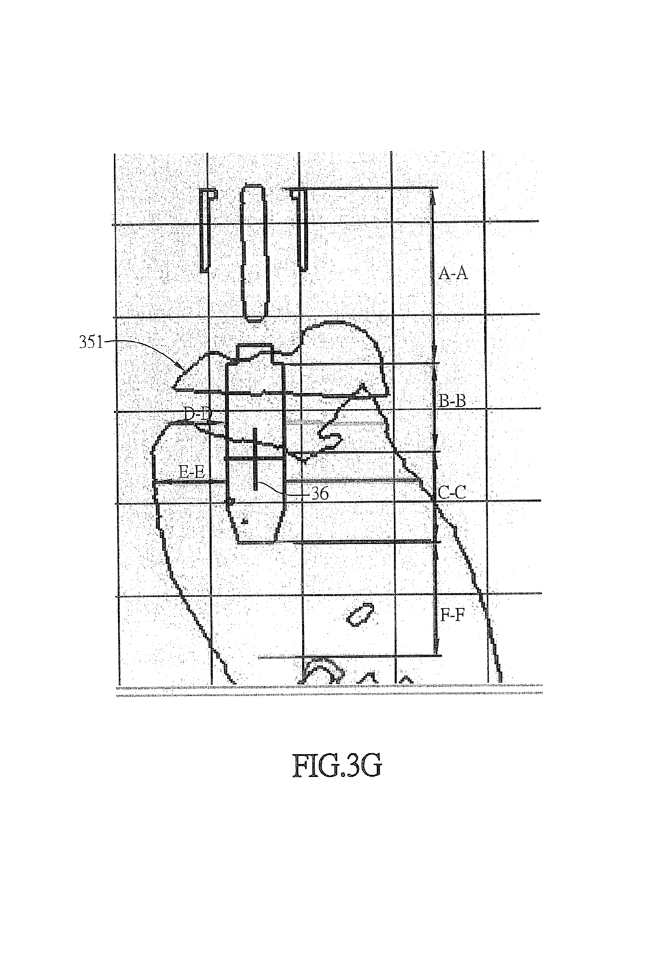

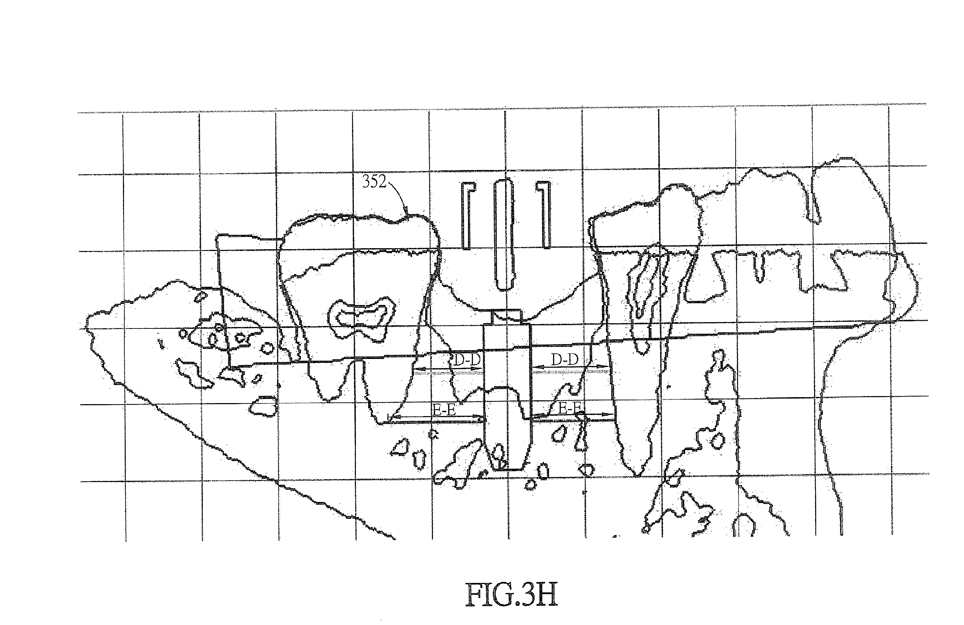

The memory module 13 also stores several calculation methods for the characteristic distances. FIG. 3G is a schematic diagram of the characteristic distances in the BL cross-section image, and FIG. 3H is a schematic diagram of the characteristic distances in the MD cross-section image. As shown in FIGS. 3G and 3H, the targets of the calculation methods stored in the memory module 13 include the distance A-A which is from the bottom of the metal ring disposed on the implantation guiding plate to the top of the implant, the distance B-B by which the implant's top is protruded from the jaw bone, the distance C-C by which the implant is embedded into the jaw bone, the distance D-D which is from the point of 1/3 length of the implant counted from the implant's top to the bone wall, adjacent tooth root or adjacent implant, the lateral distance E-E which is from the point of 2/3 length of the implant counted from the implant's top to the bone wall, adjacent tooth root or adjacent implant, or the vertical distance F-F which is from the implant's bottom to the nerve or jaw bone's wall (having no nerves). The above distances are displayed in the reference zone 23 in a way of characteristic values (as shown in the reference zone 23 of FIG. 2), and can be referred to by the user or surgeon as another reference information besides the image information. To be noted, the program 14 can further calculate the lingual, buccal, mesial and distal characteristic distances of some items (e.g. the distances D-D and E-E) and cause them to be displayed in the reference zone (as shown in FIG. 2) for providing the further reference information for the surgeon.

For the above calculation methods, the coordinates of the specific characteristic points in the BL or MD cross-section images 351 or 352 conforming to the setting are found first by the image recognition technology, and then the distance between the two characteristic points are calculated. So, when the setting is adjusted, the location of the characteristic point is adjusted accordingly. Besides, adding or eliminating some type of the characteristic distance and the characteristic value thereof is allowed. For example, one of the above six characteristic distances can be eliminated, or a new characteristic distance which is from the point of 1/2 length of the implant to some tissue can be added.

For the implant path planning, the user or surgeon can refer to the BL cross-section image 351 or the characteristic values to adjust the position of the implant image 34 relative to the jaw image 31 for avoiding the implant from contacting nerves or blood vessels. Practically, the user or surgeon can use an external input appliance, such as a mouse, to move or rotate the implant image 34 for adjusting the position or depth of the implant image 34 relative to the jaw image 31. In other embodiments, during the adjustment of the implant image 34, the relative distance between the implant body portion 341 and the metal ring portion 342 can be adjusted. In the cross-section image zone 22, the program 14 can lap an adjustment label 36 over the implant image 34, and the implant image 34 can be adjusted by moving or rotating the adjustment label 36. Besides, the program 14 also can immediately calculate the changed characteristic distance so that the characteristic value displayed in the reference zone 23 can be updated right away. Furthermore, when the implant image 34 in one of the BL cross-section image unit 221 and MD cross-section image unit 222 is adjusted, that in the other is also adjusted accordingly and immediately for the synchronous observation.

Accordingly, the user or surgeon can determine if the implant path is appropriate by the BL cross-section image 351 and further by the characteristic values in the MD cross-section image 352. Besides, the program 14 also can provide a function that the characteristic value is provided with a special visual effect, such as a highlight, when the characteristic value is less than a threshold value.

To be noted, the program 14 can determine if any characteristic value corresponding to the characteristic distance exceeds a threshold value. If any, the program 14 can cause a warning image to be displayed for the manual adjustment.

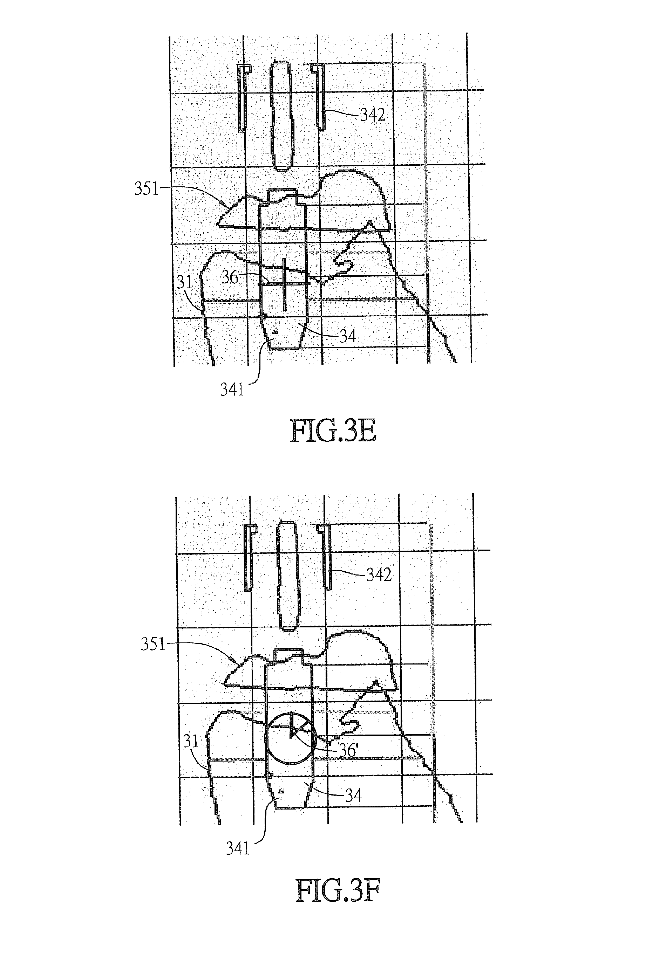

The above adjustment label 36 can be, for example, a crisscross movement adjustment label 36 as shown in FIG. 3E, or a circular rotation adjustment label 36' as shown in FIG. 3F. Literally, the crisscross movement adjustment label 36 is for moving the implant image 34 to change its depth or position relative to the jaw image 31, and the circular rotation adjustment label 36' is for adjusting the angle of the implant image 34 relative to the jaw image 31. In this embodiment, the movement adjustment label 36 and the rotation adjustment label 36' can be switched by the user or surgeon pressing or clicking the operating unit 244 in the function item zone 24 that provides a switching function.

The characteristic value also can be adjusted. Practically, the user or surgeon can use an external keyboard to directly change the characteristic value for adjusting the depth or angle of the implant image 34 relative to the jaw image 31.

Besides, in other embodiments when a plurality of the implant positions are labeled in the jaw image 31 and a plurality of the implant images are lapped over the jaw image 31 according to the implant positions, the user can first complete the implant path planning of one of the implant positions, and then when the user or surgeon presses or clicks the operating unit 241 in the function item zone 24, the program 14 starts a function of parallel adjustment whereby the other implant images are parallel to the implant image that has the implant path planning completed before. In other words, the program 14 can complete the implant path planning of the all implant positions at one time by paralleling the all implant images, and thereby the operation is facilitated. Moreover, the function item zone 24 has an operating unit 245, which can cause a pull-down menu to show up when selected or clicked. When a proper implant standard (provided by the pull-down menu for example) is selected, the program 14 will accordingly change the image and characteristic value. Of course, the step of selecting the proper implant standard can be executed before generating the BL cross-section image 351 or MD cross-section image 352. However, the invention is not limited thereto.

To be noted, in order to obtain the image or data information from other cross-section images, the user or surgeon can adjust the section L-L in the jaw image zone 21, such as clockwise or counterclockwise rotating the section L-L.

After completing the adjustment, the program 14 can output information in relation to the implant position as a planning result data, which can include the standard of the implant's dimensions, the coordinates of the opposite ends (the upper end and lower end) of the implant, or their combination, to a manufacturer or directly to an apparatus manufacturing the occlusal splint. The above coordinates can be recorded in the form of X-axis and Y-axis parameters.

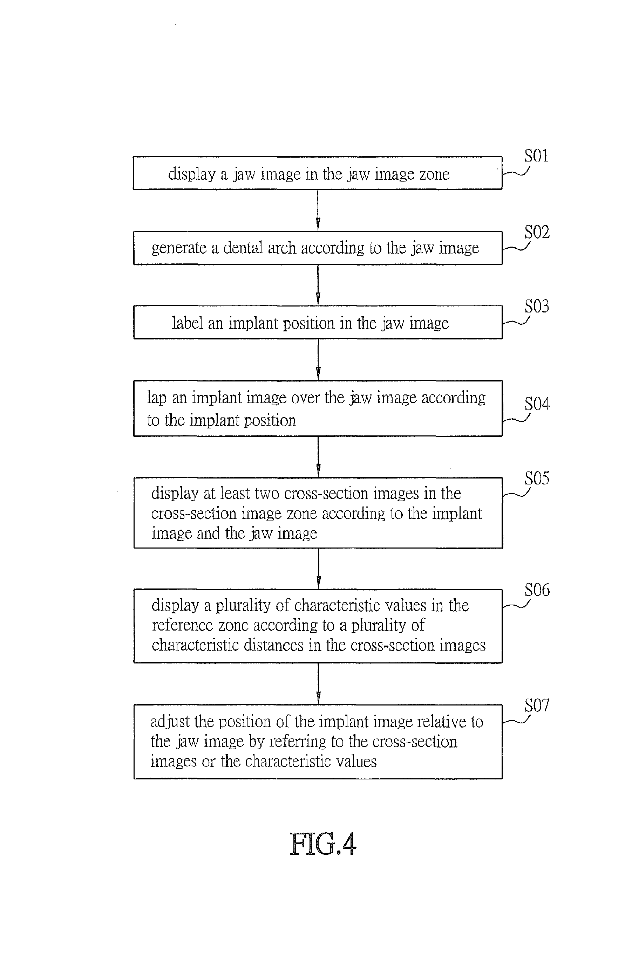

FIG. 4 is a flow chart of a method for dental implant path planning according to a preferred embodiment of the invention. The method for dental implant path planning is applied to a computer, which displays a planning interface having a jaw image zone, a cross-section image zone and a reference zone. The method for dental implant path planning includes the steps S01-S07.

The step S01 is to display a jaw image in the jaw image zone.

The step S02 is to generate a dental arch according to the jaw image.

The step S03 is to label an implant position in the jaw image.

The step S04 is to lap an implant image over the jaw image according to the implant position.

The step S05 is to display at least two cross-section images in the cross-section image zone according to the implant image and the jaw image.

The step S06 is to display a plurality of characteristic values in the reference zone according to a plurality of characteristic distances in the cross-section images.

The step S07 is to adjust the position of the implant image relative to the jaw image by referring to the cross-section images or the characteristic values.

The details and technical features of the method for dental implant path planning in this embodiment are similar to the illustration of the system for dental implant path planning of the above embodiment, and therefore they are not described here for conciseness. To be noted, in this embodiment, when the method for dental implant path planning is applied to the computer, the computer becomes the system for dental implant path planning of the above embodiment.

In summary, by the method and system for dental implant path planning according to this invention, the user or surgeon can easily implement the implant path planning with the help of the computer and program. Besides, in the method and system of this invention, both of the visualized and quantified data are generated and shown by a planning interface for reference. Thereby, the surgeon can accurately control the result of the implantation surgery before performing the surgery, and besides, the safety of the surgery can be kept.

Furthermore, the method and system of this invention can output the planning result in the form of digital data to a manufacturer of occlusal splints or an apparatus manufacturing occlusal splints, so that the manufacturing of occlusal splints is more accurate, safer and more effective.

Although the invention has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the invention.

## Claims

1. A method for dental implant path planning applied to a computer displaying a planning interface having a jaw image zone, a cross-section image zone and a reference zone, comprising steps of:
displaying a jaw image in the jaw image zone;
generating a dental arch according to the jaw image;
labeling an implant position in the jaw image;
lapping an implant image over the jaw image according to the implant position;
displaying at least two cross-section images in the cross-section image zone according to the implant image and the jaw image;
displaying a plurality of characteristic values in the reference zone according to a plurality of characteristic distances in the cross-section images; and
adjusting the position of the implant image relative to the jaw image by referring to the cross-section images or the characteristic values.

2. The method for dental implant path planning as recited in claim 1, before the step of lapping an implant image over the jaw image according to the implant position, further comprising a step of:
generating the implant image by selecting from a plurality of default implant modules or by setting a plurality of implant parameters.

3. The method for dental implant path planning as recited in claim 1, wherein the cross-section images are taken along the section that passes through the central axis of the implant image and is perpendicular to the tangent line of the dental arch at the implant position.

4. The method for dental implant path planning as recited in claim 1, wherein an adjustment label is lapped over the implant image, and when the adjustment label is adjusted, the implant image is adjusted accordingly.

5. The method for dental implant path planning as recited in claim 1, wherein during adjusting the implant image, the implant image is moved or rotated for adjusting the depth or angle of the implant image relative to the jaw image, or for adjusting a relative distance between a implant body portion of the implant image and a metal ring portion on an implantation guiding plate.

6. The method for dental implant path planning as recited in claim 5, further comprising a step of:
changing the characteristic values in the reference zone according to the adjustment of the implant image.

7. The method for dental implant path planning as recited in claim 1, wherein the characteristic distances include the distance which is from the bottom of a metal ring disposed on an implantation guiding plate to the top of the implant, the distance by which the implant's top is protruded from the jaw bone, the distance by which the implant is embedded into the jaw bone, the distance which is from the point of 1/3 length of the implant counted from the implant's top to the bone wall, adjacent tooth root or adjacent implant, the distance which is from the point of 2/3 length of the implant counted from the implant's top to the bone wall, adjacent tooth root or adjacent implant, or the distance which is from the implant's bottom to the nerve or jaw bone's wall.

8. The method for dental implant path planning as recited in claim 1, further comprising a step of:
outputting the implant position as a planning result data.

9. The method for dental implant path planning as recited in claim 8, wherein the planning result data include the standard of the implant's dimensions, the coordinates of the opposite ends of the implant, or their combination.

10. The method for dental implant path planning as recited in claim 1, further comprising a step of:
adjusting the characteristic values for adjusting the depth or angle of the implant image relative to the jaw image, or for adjusting a relative distance between a implant body portion of the implant image and a metal ring portion on an implantation guiding plate.

11. The method for dental implant path planning as recited in claim 1, wherein the implant image includes a metal ring portion, and the central axis of the metal ring portion lies in the direction of the central axis of the implant image.

12. The method for dental implant path planning as recited in claim 1, wherein a plurality of the implant positions are labeled in the jaw image and a plurality of the implant images are lapped over the jaw image, further comprising a step of:
paralleling the implant images.

13. A system for dental implant path planning, comprising:
a display module;
a processing module;
a memory module; and
at least a program stored in the memory module and executed by the processing module, and comprising:
a command for displaying a jaw image in a jaw image zone;
a command for generating a dental arch according to the jaw image;
a command for labeling an implant position in the jaw image;
a command for lapping an implant image over the jaw image according to the implant position;
a command for displaying at least two cross-section images in a cross-section image zone according to the implant image and the jaw image;
a command for displaying a plurality of characteristic values in a reference zone according to a plurality of characteristic distances in the cross-section images; and
a command for adjusting the position of the implant image relative to the jaw image by referring to the cross-section images or the characteristic values.

14. The system for dental implant path planning as recited in claim 13, where before the command for lapping an implant image over the jaw image according to the implant position, the program further comprises:
a command for generating the implant image by selecting from a plurality of default implant modules or by setting a plurality of implant parameters.

15. The system for dental implant path planning as recited in claim 13, where the program further comprises:
a command of adjusting the characteristic values for adjusting the depth or angle of the implant image relative to the jaw image, or for adjusting a relative distance between a implant body portion of the implant image and a metal ring portion on an implantation guiding plate.
